# EUROPEAN PATENT APPLICATION

(11) **EP 3 269 801 A1**
(43) Date of publication of application: **17.01.2018**
(21) Application number: 15883641.1
(22) Date of filing: 17.03.2015
(51) Int. Cl.: C12N 5/071, C12N 5/02, C12Q 1/02, G01N 33/50

(54) **METHOD FOR IMPROVING DRUG METABOLISM FUNCTION OF HUMAN STEM CELL-DERIVED HEPATOCYTE**

(30) Priority: 12.03.2015 KR 20150034270; 12.03.2015 KR 20150034271
(71) Applicant: Korea Advanced Institute of Science and Technology, Daejeon 305-338 (KR)
(72) Inventor: JEONG, Won-il, Daejeon 305-338 (KR); HAN, Yong-Mahn, Daejeon 305-707 (KR); KIM, Mi Young, Daejeon 305-338 (KR); EUN, Hyuk Soo, Daejeon 305-338 (KR); JANG, Mi-Jin, Daejeon 305-338 (KR)
(74) Representative: Forstmeyer, Dietmar
(86) International application number: PCT/KR2015/002576
(87) International publication number: WO 2016/143931

(57) **Abstract**

The present invention relates to a method for improving drug metabolism function of human stem cell-derived hepatocytes. More precisely, the human stem cell-derived hepatocytes are similar in cell morphology to human hepatocytes but display reduced expressions of drug or alcohol metabolism associated enzymes and antioxidant enzymes. So, the inventors co-cultured the hepatocytes differentiated from human stem cells with mouse hepatic stellate cells. As a result, it was confirmed that alcohol mediated toxicity was reduced so that liver cell damage or apoptosis level was reduced. In the meantime, the expressions of drug and alcohol metabolism associated enzymes and antioxidant enzymes were increased. It was also confirmed the changes in the expression pattern of retinol associated genes. The present inventors further co-cultured the hepatocytes differentiated from human stem cells with hepatic stellate cells separated from the mouse having disorder in retinol metabolism to retinoic acid. As a result, it was confirmed that liver cell damage was not reduced and the expressions of enzymes involved in drug and alcohol metabolism and antioxidant enzymes were not changed. When the hepatocytes differentiated from human stem cells were treated with retinoic acid, the expressions of enzymes involved in drug and alcohol metabolism and antioxidant enzymes were increased but apoptosis was decreased. Therefore, the method of co-culturing the hepatocytes differentiated from human stem cells above with hepatic stellate cells and the method for the treatment of retinoic acid to the hepatocytes differentiated from human stem cells were confirmed to be efficiently used as methods for improving drug metabolism function of the human stem cell-derived hepatocytes.

<110>	Korea Advanced Institute of Science and Technology

<120>	A method for improvement of drug metabolizing function of human
	stem cell-derived hepatocytes

<130>	2015FP0-02-002_PCT

<150>	KR 10-2015-0034270
<151>	2015-03-12

<150>	KR 10-2015-0034271
<151>	2015-03-12

<160>	36

<170>	KopatentIn 2.0

<210>	1
<211>	21
<212>	DNA
<213>	Artificial Sequence

<220>
<223>	ADH1a_F

<400>	1
agtcatccca ctcgctattc c	21

<210>	2
<211>	21
<212>	DNA
<213>	Artificial Sequence

<220>
<223>	ADH1a_R

<400>	2
gtcccctgag gattgcttac a	21

<210>	3
<211>	19
<212>	DNA
<213>	Artificial Sequence

<220>
<223>	ADH1b_F

<400>	3
cccggagagc aactactgc	19

<210>	4
<211>	21
<212>	DNA
<213>	Artificial Sequence

<220>
<223>	ADH1b_R

<400>	4
aaccagtcga gaatccacag c	21

<210>	5
<211>	19
<212>	DNA
<213>	Artificial Sequence

<220>
<223>	ADH1c_F

<400>	5
ctcgcccctg gagaaagtc	19

<210>	6
<211>	19
<212>	DNA
<213>	Artificial Sequence

<220>
<223>	ADH1c_R

<400>	6
ggcccccaac tctttagcc	19

<210>	7
<211>	18
<212>	DNA
<213>	Artificial Sequence

<220>
<223>	ALDH2_F

<400>	7
tcggctacat caacacgg	18

<210>	8
<211>	19
<212>	DNA
<213>	Artificial Sequence

<220>
<223>	ALDH2_R

<400>	8
tctcccaaca acctcctct	19

<210>	9
<211>	21
<212>	DNA
<213>	Artificial Sequence

<220>
<223>	CYP2E1_F

<400>	9
gcaagagatg ccctacatgg a	21

<210>	10
<211>	18

<212>	DNA
<213>	Artificial Sequence

<220>
<223>	CYP2E1_R


<400>	10
gggcacgagg gtgatgaa	18


<210>	11
<211>	22
<212>	DNA
<213>	Artificial Sequence

<220>
<223>	CYP2D6_F


<400>	11
ctaagggaac gacactcatc ac	22


<210>	12
<211>	22
<212>	DNA
<213>	Artificial Sequence

<220>
<223>	CYP2D6_R


<400>	12
ctcaccagga aagcaaagac ac	22


<210>	13
<211>	23
<212>	DNA
<213>	Artificial Sequence

<220>
<223>	CYP3A4_F


<400>	13
caggaggaaa ttgatgcagt ttt	23


<210>	14
<211>	27
<212>	DNA
<213>	Artificial Sequence

<220>
<223>	CYP3A4_R


<400>	14
gtcaagatac tccatctgta gcacagt	27


<210>	15
<211>	24
<212>	DNA
<213>	Artificial Sequence

<220>
<223>    Catalase_F


<400>    15
gttactcagg tgcgggcatt ctat                                              24


<210>    16
<211>    24
<212>    DNA
<213>    Artificial Sequence

<220>
<223>    Catalase_R


<400>    16
gaagttcttg accgctttct tctg                                              24


<210>    17
<211>    24
<212>    DNA
<213>    Artificial Sequence

<220>
<223>    Mn-SOD_F


<400>    17
ctccccgacc tgccctacga ctac                                              24


<210>    18
<211>    24
<212>    DNA
<213>    Artificial Sequence

<220>
<223>    Mn-SOD_R


<400>    18
aaaccaagcc aaccccaacc tgag                                              24


<210>    19
<211>    20
<212>    DNA
<213>    Artificial Sequence

<220>
<223>    b-actin_F


<400>    19
agcgagcatc ccccaaagtt                                                   20


<210>    20
<211>    20
<212>    DNA
<213>    Artificial Sequence

<220>
<223>    b-actin_R

<400>    20
gggcacgaag gctcatcatt                                                    20

<210>    21
<211>    20
<212>    DNA
<213>    Artificial Sequence

<220>
<223>    B-actin_F

<400>    21
agagggaaat cgtgcgtgac                                                    20

<210>    22
<211>    21
<212>    DNA
<213>    Artificial Sequence

<220>
<223>    B-actin_R

<400>    22
caatagtgat gacctggccg t                                                  21

<210>    23
<211>    25
<212>    DNA
<213>    Artificial Sequence

<220>
<223>    LRAT_F

<400>    23
ctgaccaatg acaaggaacg cactc                                              25

<210>    24
<211>    26
<212>    DNA
<213>    Artificial Sequence

<220>
<223>    LRAT_R

<400>    24
ctaatcccaa gacagccgaa gcaaga                                             26

<210>    25
<211>    20
<212>    DNA
<213>    Artificial Sequence

<220>
<223>    ADH3_F

<400>    25
gtgggagtag ctgcttcagg                                                    20

<210> 26
<211> 21
<212> DNA
<213> Artificial Sequence

<220>
<223> ADH3_R

<400> 26
gtgcatcaga tcaaaggctt g 21

<210> 27
<211> 20
<212> DNA
<213> Artificial Sequence

<220>
<223> ALDH1A1_F

<400> 27
gctcaagaca gtcgcaatga 20

<210> 28
<211> 21
<212> DNA
<213> Artificial Sequence

<220>
<223> ALDH1A1_R

<400> 28
tggataagaa ctggggtcac a 21

<210> 29
<211> 20
<212> DNA
<213> Artificial Sequence

<220>
<223> Rbp1_F

<400> 29
cagtgtgtgc agaagggaga 20

<210> 30
<211> 20
<212> DNA
<213> Artificial Sequence

<220>
<223> Rbp1_R

<400> 30
aggttatctc ctcgggctgt 20

<210> 31

<211>	20
<212>	DNA
<213>	Artificial Sequence

<220>
<223>	Crabp1_F

<400>	31
cttactggac ccgagagctg	20

<210>	32
<211>	20
<212>	DNA
<213>	Artificial Sequence

<220>
<223>	Crabp1_R

<400>	32
ggaaaatggg gttgcctaat	20

<210>	33
<211>	20
<212>	DNA
<213>	Artificial Sequence

<220>
<223>	HGF_F

<400>	33
ttcccagctg gtctatggtc	20

<210>	34
<211>	20
<212>	DNA
<213>	Artificial Sequence

<220>
<223>	HGF_R

<400>	34
tggtgctgac tgcatttctc	20

<210>	35
<211>	22
<212>	DNA
<213>	Artificial Sequence

<220>
<223>	IGF-1_F

<400>	35
cacatcatgt cgtcttcaca cc	22

<210>	36
<211>	23
<212>	DNA
<213>	Artificial Sequence

<220>
<223>    IGF-1_R

<400>    36
ggaagcaaca ctcatccaca atg                                                23

## Description

### BACKGROUND OF THE INVENTION

### 1. Field of the Invention

The present invention relates to a method for the improvement of drug metabolism of human stem cell-derived hepatocytes comprising the step of co-culturing the hepatocytes differentiated from human stem cells with hepatic stellate cells (HSCs) or the step of treating the human stem cell-derived hepatocytes with retinoic acid.

### 2. Description of the Related Art

The increase of chronic alcoholic patients due to the stress originated from the complicated modern social life and from many different sources has been a big issue not only in Western advanced countries but also world-widely. Alcohol is mostly metabolized in the liver. However, the liver metabolism for alcohol is limited. When it goes beyond the limit, it causes various metabolic disorders. That is, in the cells of a chronic alcoholic, the ratio of NADH/NAD⁺ is increased to cause carbohydrate, protein, and lipid metabolic disorders. In particular, the disorder of lipid metabolism in the liver tissue results in the inhibition of fatty acid oxidation but in the increase of fatty acid synthesis. Also, the microtubule is damaged by the toxic acetaldehyde, which causes fatty liver and if it progresses alcoholic hepatitis or liver cirrhosis is developed.

The biggest problem in the liver transplantation to treat liver disease is the lack of a donor. Recently, cell therapy using hepatocytes is rising as an alternative to over-come the problem of few numbers of a donor for the liver transplantation. Accordingly, human embryonic stem cells that are able to be proliferated infinitely are drawing our attention as a promising material to supply enough hepatocytes useful for the treatment of liver disease. However, there are still problems for the hepatocytes differentiated from human embryonic stem cells to be used as a material for cell therapy to treat liver disease. First, it is very difficult to produce *in vitro* hepatocytes having a completely normal, exactly same liver functions as the original cells because the technique to induce the differentiation is not completed owing to the short knowledge on the exact mechanism of the entire human embryogenesis process. Second, the survival rate of the human embryonic stem cell-derived hepatocytes in the course of the transplantation in a liver disease animal model is very low because of the reason described above, so that the damaged liver functions cannot be fully and efficiently recovered. Third, there might be side effects such as the generation of teratoma etc because of the non-differentiated stem cells remaining after the inducement of the differentiation. In conclusion, the human embryonic stem cell-derived hepatocytes are still limited in use for cell therapy for liver diseases.

To solve the problems described above, the present inventors made many attempts and as a result confirmed that human stem cell-derived hepatocytes were similar in shape with the human liver cells but displayed the decrease of enzymes involved in drug or alcohol metabolism and the down-regulation of antioxidant enzymes therein. The present inventors thereafter co-cultured human stem cell-derived hepatocytes with hepatic stellate cells (HSCs). As a result, toxicity caused by alcohol was reduced and thus liver cell damage or apoptosis was also decreased. In addition, the expressions of enzymes involved in drug and alcohol metabolism and antioxidant enzymes were all increased. The present inventors also co-cultured human stem cell-derived hepatocytes with mouse hepatic stellate cells. As a result, the inventors confirmed that the retinol related gene expression pattern was changed. The present inventors further co-cultured the hepatocytes differentiated from human stem cells with the hepatic stellate cells separated from the mouse having disorder in retinol metabolism to retinoic acid. As a result, it was confirmed that liver cell damage was not reduced and the expressions of enzymes involved in drug and alcohol metabolism and antioxidant enzymes were not changed. When the human stem cell-derived hepatocytes were treated with retinoic acid, the expressions of enzymes involved in drug and alcohol metabolism and antioxidant enzymes were increased but apoptosis was decreased. Therefore, the present inventors completed this invention by confirming that the method for co-culturing human stem cell-derived hepatocytes with hepatic stellate cells and the method for the treatment of retinoic acid to the human stem cell-derived hepatocytes could be useful methods to improve drug metabolism function of human stem cell-derived hepatocytes.

### SUMMARY OF THE INVENTION

It is an object of the present invention to provide a method for improving drug metabolism function of human stem cell-derived hepatocytes comprising the step of co-culturing the hepatocytes differentiated from human stem cells with hepatic stellate cells (HSCs).

It is another object of the present invention to provide a method for improving drug metabolism function of human stem cell-derived hepatocytes comprising the step of treating the hepatocytes differentiated from human stem cells with retinoic acid.

It is also an object of the present invention to provide human stem cell-derived hepatocytes prepared by the method above.

To achieve the above objects, the present invention provides a method for improving drug metabolism function of human stem cell-derived hepatocytes comprising the step of co-culturing the hepatocytes differentiated from human stem cells with hepatic stellate cells (HSCs).

The present invention also provides a method for improving drug metabolism function of human stem cell-derived hepatocytes comprising the step of treating the hepatocytes differentiated from human stem cells with retinoic acid *ex vivo.*

The present invention also provides human stem cell-derived hepatocytes prepared by the method above.

The present invention further provides a method for evaluating drug or alcohol metabolism in hepatocytes comprising the following steps:
1) treating the said hepatocytes above with a test drug or alcohol *ex vivo;*
2) measuring the expression level of the enzyme related to the drug or alcohol metabolism in the hepatocytes of step 1); and
3) evaluating the drug or alcohol metabolism level by the expression level of the enzyme related to the drug or alcohol metabolism measured in step 2).

The present invention also provides an evaluation system of drug or alcohol metabolism comprising the hepatocytes above.

In addition, the present invention provides the hepatocytes above in order to use the cells for the evaluation system of drug or alcohol metabolism.

### ADVANTAGEOUS EFFECT

The present invention relates to a method for improving drug metabolism function of human stem cell-derived hepatocytes. More precisely, the human stem cell-derived hepatocytes are similar in cell morphology to human hepatocytes but display reduced expressions of drug or alcohol metabolism associated enzymes and antioxidant enzymes. So, the inventors co-cultured the hepatocytes differentiated from human stem cells with mouse hepatic stellate cells. As a result, it was confirmed that alcohol mediated toxicity was reduced so that liver cell damage or apoptosis level was reduced. In the meantime, the expressions of drug and alcohol metabolism associated enzymes and antioxidant enzymes were increased. It was also confirmed that the changes in the expression pattern of retinol associated genes were made. The present inventors further co-cultured the hepatocytes differentiated from human stem cells with the hepatic stellate cells separated from the mouse having disorder in retinol metabolism to retinoic acid. As a result, it was confirmed that liver cell damage was not reduced and the expressions of enzymes involved in drug and alcohol metabolism and antioxidant enzymes were not changed. When the hepatocytes differentiated from human stem cells were treated with retinoic acid, the expressions of enzymes involved in drug and alcohol metabolism and antioxidant enzymes were increased but apoptosis was decreased. Therefore, the method of co-culturing the hepatocytes differentiated from human stem cells above with hepatic stellate cells and the method for the treatment of retinoic acid to the hepatocytes differentiated from human stem cells were confirmed to be efficiently used as methods for improving drug metabolism function of the human stem cell-derived hepatocytes.

### BRIEF DESCRIPTION OF THE DRAWINGS

The application of the preferred embodiments of the present invention is best understood with reference to the accompanying drawings, wherein:
Figure 1 is a diagram illustrating the morphology of human embryonic stem cell-derived hepatocytes (hES-Hep) and human hepatocytes (Human-Hep).
Figure 2 is a diagram illustrating the comparison of gene expression patterns between adult hepatocytes (adult-hep) and human embryonic stem cell-derived hepatocytes (hES-Hep).
Figure 3 is a diagram illustrating the comparison of gene expression patterns between adult-Hep and Fetal-Hep and the comparison of gene expression patterns between Fetal liver and hES-Hep.
Figure 4 is a diagram illustrating the method of co-culturing human embryonic stem cell-derived hepatocytes (hES-Hep) with mouse hepatic stellate cells (mHSCs).
Figure 5 is a diagram illustrating the liver cell damage observed after the co-culture of human embryonic stem cell-derived hepatocytes (hES-Hep) with mouse hepatic stellate cells (mHSCs).
Figure 6 is a diagram illustrating the apoptosis observed after the co-culture of human embryonic stem cell-derived hepatocytes (hES-Hep) with mouse hepatic stellate cells (mHSCs).
Figure 7 is a diagram illustrating the gene expressions of alcohol and drug metabolism associated enzymes and antioxidant enzymes after the co-culture of human embryonic stem cell-derived hepatocytes (hES-Hep) with mouse hepatic stellate cells (mHSCs).
Figure 8 is a diagram illustrating the protein expressions of alcohol and drug metabolism associated enzymes and antioxidant enzymes after the co-culture of human embryonic stem cell-derived hepatocytes (hES-Hep) with mouse hepatic stellate cells (mHSCs).
Figure 9 is a diagram illustrating the expression of CYP2E1 after the co-culture of human embryonic stem cell-derived hepatocytes (hES-Hep) with mouse hepatic stellate cells (mHSCs).
Figure 10 is a diagram illustrating the antioxidant enzyme activity observed after the co-culture of human embryonic stem cell-derived hepatocytes (hES-Hep) with mouse hepatic stellate cells (mHSCs).
Figure 11 is a diagram illustrating the gene expression in mHSCs observed after the co-culture of human embryonic stem cell-derived hepatocytes (hES-Hep) with mouse hepatic stellate cells (mHSCs).
Figure 12 is a diagram illustrating the liver cell damage observed after the co-culture of human embryonic stem cell-derived hepatocytes (hES-Hep) with the mouse hepatic stellate cells (mHSCs) separated from the liver of a wild type (WT) mouse and a genetically engineered mouse with retinoic acid metabolism deficiency (Raldh1^{-/-}) so that it could not metabolize retinol into retinoic acid.
Figure 13 is a diagram illustrating the expressions of alcohol and drug metabolism associated enzymes and antioxidant enzymes observed after the co-culture of human embryonic stem cell-derived hepatocytes (hES-Hep) with the mouse hepatic stellate cells (mHSCs) separated from the liver of a wild type (WT) mouse and a genetically engineered mouse with retinoic acid metabolism deficiency (Raldh1^{-/-}) so that it could not metabolize retinol into retinoic acid.
Figure 14 is a diagram illustrating the protein expressions of alcohol and drug metabolism associated enzymes and antioxidant enzymes observed after the co-culture of human embryonic stem cell-derived hepatocytes (hES-Hep) with the mouse hepatic stellate cells (mHSCs) separated from the liver of a wild type (WT) mouse and a genetically engineered mouse with retinoic acid metabolism deficiency (Raldh1^{-/-}) so that it could not metabolize retinol into retinoic acid.
Figure 15 is a diagram illustrating the retinol related gene expression observed after the co-culture of human embryonic stem cell-derived hepatocytes (hES-Hep) with the mouse hepatic stellate cells (mHSCs) separated from the liver of a wild type (WT) mouse and a genetically engineered mouse with retinoic acid metabolism deficiency (Raldh1^{-/-}) so that it could not metabolize retinol into retinoic acid.
Figure 16 is a diagram illustrating the expressions of alcohol and drug metabolism associated enzymes and antioxidant enzymes after the treatment of retinoic acid to human embryonic stem cell-derived hepatocytes (hES-Hep).
Figure 17 is a diagram illustrating the expressions of alcohol and drug metabolism associated enzymes and antioxidant enzymes after the treatment of retinoic acid and ethanol to human embryonic stem cell-derived hepatocytes (hES-Hep).
Figure 18 is a diagram illustrating the expression of CYP2E1 observed after the treatment of retinoic acid to human embryonic stem cell-derived hepatocytes (hES-Hep).
Figure 19 is a diagram illustrating the apoptosis observed after the treatment of retinoic acid to human embryonic stem cell-derived hepatocytes (hES-Hep).

### DESCRIPTION OF THE PREFERRED EMBODIMENTS

Hereinafter, the present invention is described in detail.

The present invention provides a method for improving drug metabolism function of human stem cell-derived hepatocytes comprising the step of co-culturing the hepatocytes differentiated from human stem cells with hepatic stellate cells (HSCs) *ex vivo.*

In the method above, the hepatocytes are preferably differentiated by the following steps, but not always limited thereto:
1) culturing human stem cells to induce the differentiation; and
2) culturing the differentiated cells above in a medium supplemented with hepatocyte growth factor.

In the method above, the human stem cells are preferably human embryonic stem cells or induced pluripotent stem cells (iPS cells), but not always limited thereto.

In the method above, the hepatic stellate cells are preferably mouse originated hepatic stellate cells, but not always limited thereto.

In the method above, the ratio of hepatic stellate cells to human stem cell-derived hepatocytes for the co-culture is preferably 1:2, but not always limited thereto.

In the method above, it is preferred in the cultured hepatocytes that the expressions of alcohol or drug metabolism associated enzymes and antioxidant enzymes are increased. At this time, the alcohol metabolism associated enzyme is one or more enzymes selected from the group consisting of ADH1a, ADH1b, ADH1c, and CYP2E1, and the drug metabolism associated enzyme is one or more enzymes selected from the group consisting of CYP2C9, CYP2D6, and CYP3A4, and the antioxidant enzyme is either or both of catalase and Mn-SOD, but not always limited thereto.

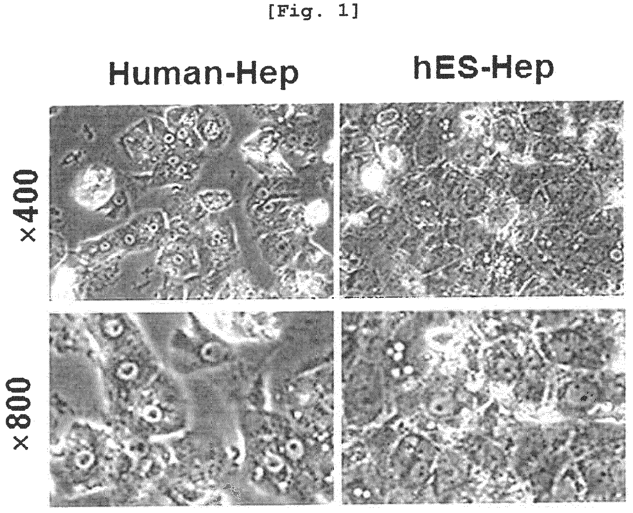

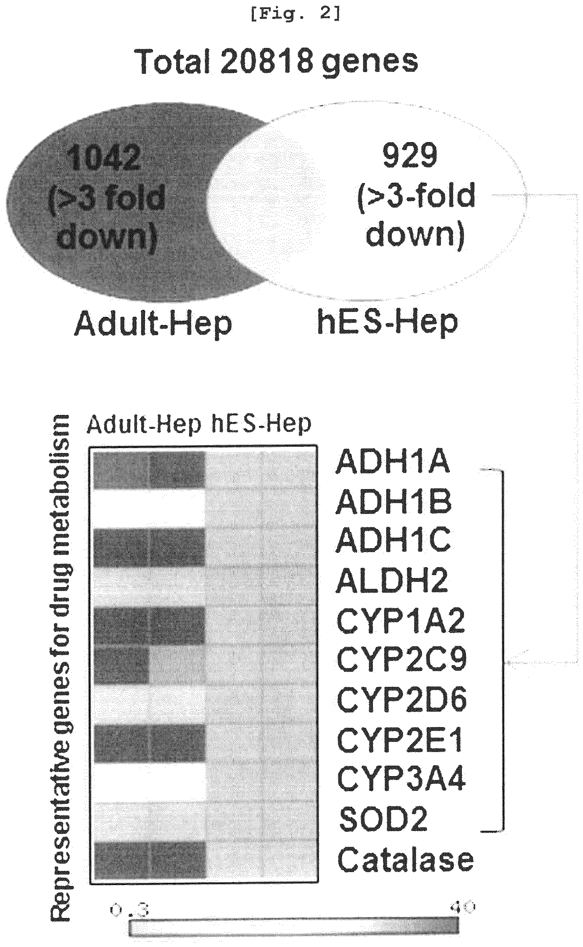

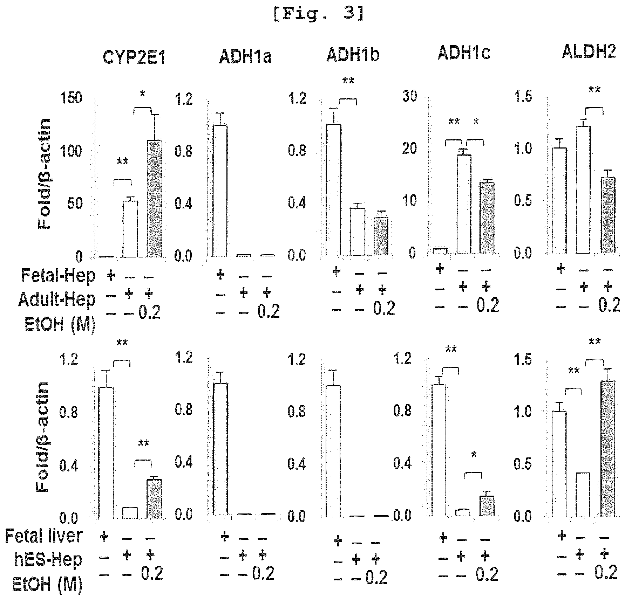

In a preferred embodiment of the present invention, the present inventors induced the differentiation of human embryonic stem cells into hepatocytes. Then, the inventors compared the differentiated hepatocytes originated from human embryonic stem cells with human hepatocytes. As a result, it was confirmed that the hepatocytes differentiated from human embryonic stem cells were similar in cell shape (see Figure 1) but displayed a different gene expression pattern. Precisely, the expression of drug or alcohol metabolism associated gene and anti-oxidation associated gene was significantly reduced (see Figure 2). The inventors also compared the gene expressions between adult hepatocytes (Adult-Hep) and fetal hepatocytes (Fetal-Hep) and between Fetal liver and human embryonic stem cell-derived hepatocytes (hES-Hep). As a result, the expressions of those genes associated with alcohol metabolism were reduced in Fetal liver, compared with adult hepatocytes, and the expressions of those genes associated with alcohol metabolism were reduced more significantly in human embryonic stem cell-derived hepatocytes (hES-Hep) than in Fetal liver (see Figure 3).

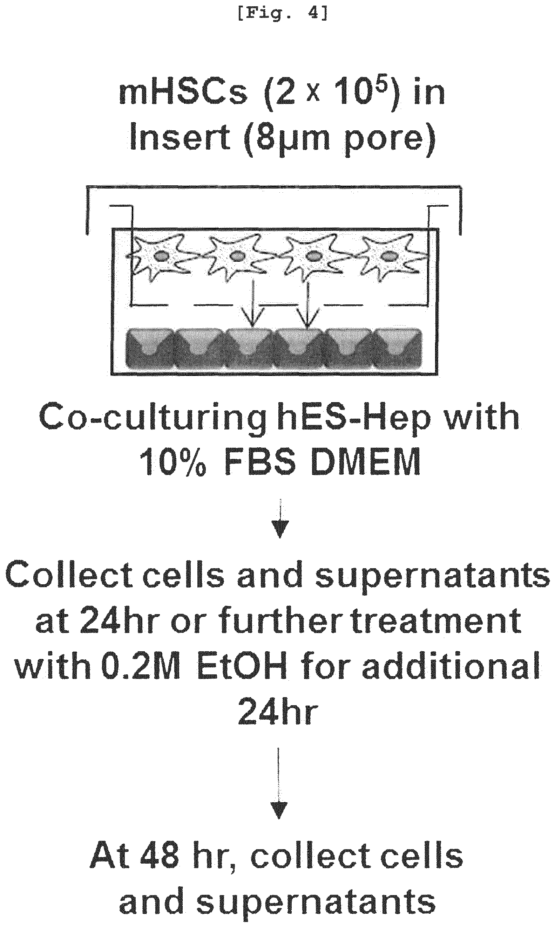

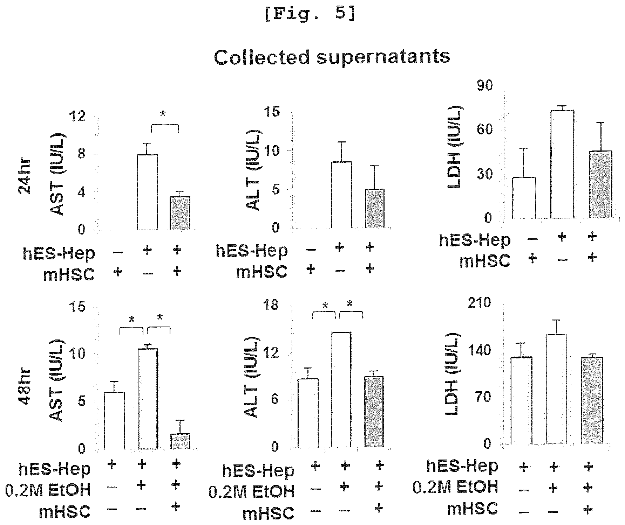

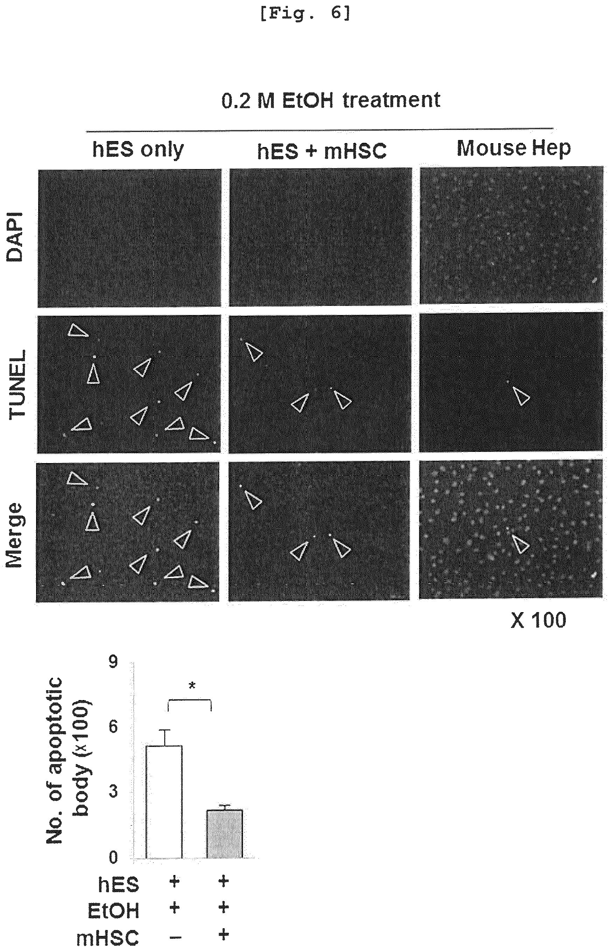

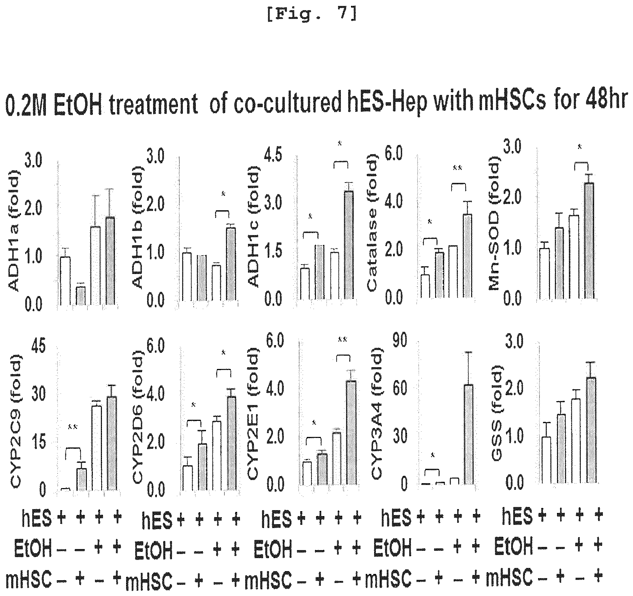

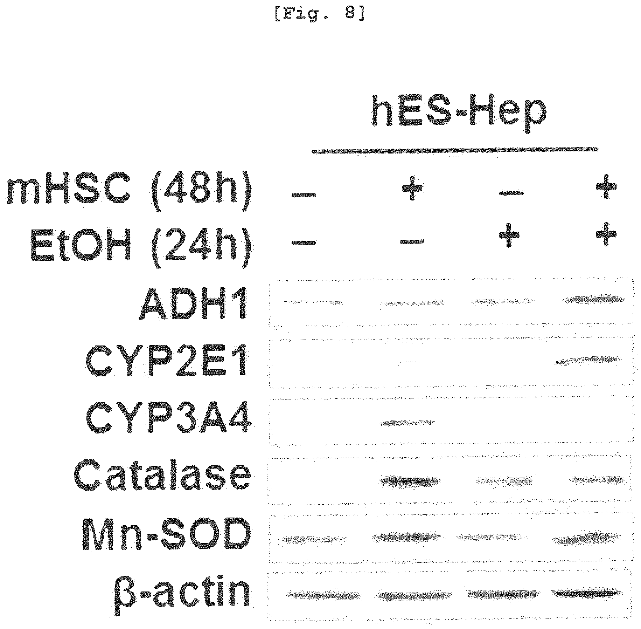

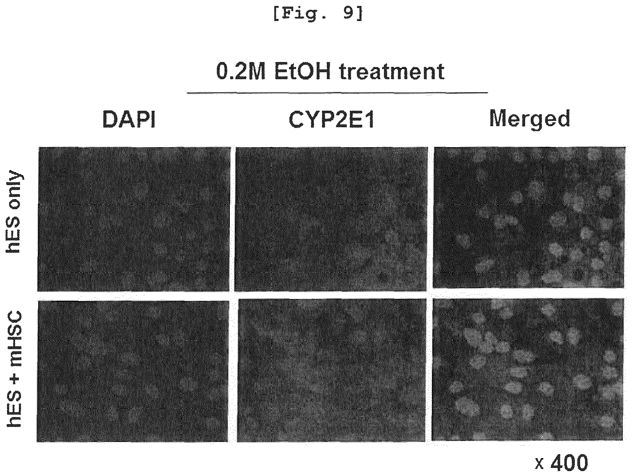

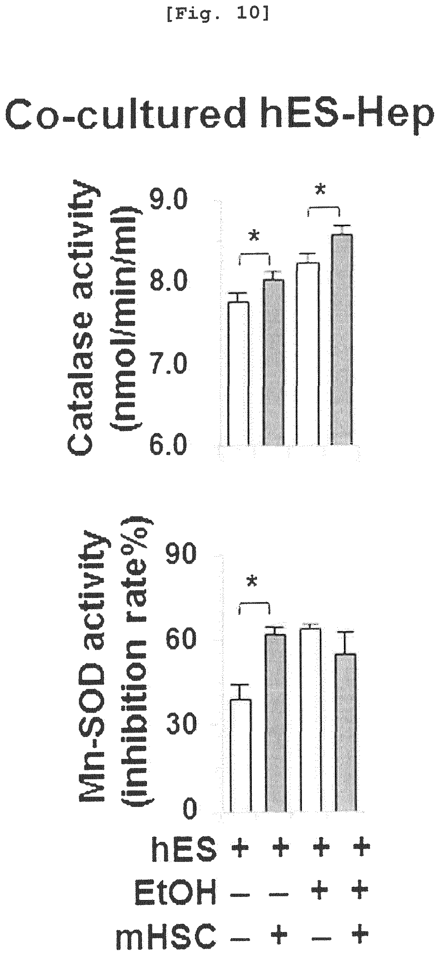

Mouse hepatic stellate cells were co-cultured with human embryonic stem cell-derived hepatocytes (see Figure 4). Then, liver cell damage was observed. As a result, simply with the co-culture for 24 hours, the levels of AST, ALT, and LDH were decreased. The levels of AST, ALT, and LDH were also decreased by the treatment of alcohol (ethanol) (see Figure 5). Apoptosis was also observed. As a result, apoptosis in human embryonic stem cell-derived hepatocytes (hES-Hep) was increased by the treatment of alcohol, but was significantly decreased in the human embryonic stem cell-derived hepatocytes (hES-Hep) co-cultured with mouse hepatic stellate cells (see Figure 6). The expression pattern of alcohol metabolism associated enzyme, drug metabolism associated enzyme, and antioxidant enzyme was also investigated (see Figures 7 and 8). As a result, the expressions of those genes and proteins were increased. The expression of CYP2E1, the enzyme associated with alcohol metabolism, was investigated by immunofluorescence method. As a result, the expression of CYP2E1 was significantly increased in the human embryonic stem cell-derived hepatocytes (hES-Hep) co-cultured with mouse hepatic stellate cells (see Figure 9). The activities of catalase and Mn-SOD, the antioxidant enzymes, were investigated. As a result, the activities of catalase and Mn-SOD were increased in the human embryonic stem cell-derived hepatocytes (hES-Hep) co-cultured with mouse hepatic stellate cells (see Figure 10).

From the results of the investigation above, it was confirmed that the co-culture of the hepatocytes differentiated from human embryonic stem cells with mouse hepatic stellate cells was effective in increasing the expressions of drug metabolism and alcohol metabolism associated enzymes in human embryonic stem cell-derived hepatocytes.

Therefore, the human stem cell-derived hepatocytes of the present invention were confirmed to be similar in shape to human hepatocytes but displayed reduced expressions of drug or alcohol metabolism associated enzymes and antioxidant enzymes. Also from the result of the co-culture of human stem cell-derived hepatocytes with mouse hepatic stellate cells, it was confirmed that the alcohol dependent toxicity was reduced so that liver cell damage or apoptosis was reduced but the expressions of drug and alcohol metabolism associated enzymes and antioxidant enzymes were increased. So, the co-culture of human stem cell-derived hepatocytes with hepatic stellate cells could be used as an efficient method for improving drug metabolism function of human stem cell-derived hepatocytes.

The present invention also provides the human stem cell-derived hepatocytes prepared by the method above.

The present invention further provides a method for evaluating drug or alcohol metabolism in hepatocytes comprising the following steps:
1) treating the said hepatocytes above with a test drug or alcohol *ex vivo;*
2) measuring the expression level of the enzyme related to the drug or alcohol metabolism in the hepatocytes of step 1); and
3) evaluating the drug or alcohol metabolism level by the expression level of the enzyme related to the drug or alcohol metabolism measured in step 2).

The human stem cells above are preferably human embryonic stem cells or somatic cell derived induced pluripotent stem cells (iPS cells), but not always limited thereto.

In the method above, it is preferred in the hepatocytes that the expressions of alcohol or drug metabolism associated enzymes and antioxidant enzymes are increased. At this time, the alcohol metabolism associated enzyme is one or more enzymes selected from the group consisting of ADH1a, ADH1b, ADH1c, and CYP2E1, and the drug metabolism associated enzyme is one or more enzymes selected from the group consisting of CYP2C9, CYP2D6, and CYP3A4, and the antioxidant enzyme is either or both of catalase and Mn-SOD, but not always limited thereto.

The test drug or alcohol above is preferably selected from the group consisting of ethanol, CCl₄, and acetaminophen, but not always limited thereto.

The human stem cell-derived hepatocytes of the present invention were confirmed to be similar in shape to human hepatocytes but displayed reduced expressions of drug or alcohol metabolism associated enzymes and antioxidant enzymes. Also from the result of the co-culture of human stem cell-derived hepatocytes with mouse hepatic stellate cells, it was confirmed that the alcohol dependent toxicity was reduced so that liver cell damage or apoptosis was reduced but the expressions of drug and alcohol metabolism associated enzymes and antioxidant enzymes were increased. Therefore, the human stem cell-derived hepatocytes co-cultured with hepatic stellate cells could be efficiently used for the evaluation of drug or alcohol metabolism.

The present invention also provides an evaluation system of drug or alcohol metabolism comprising the hepatocytes above.

The present invention also provides the hepatocytes above for the evaluation system of drug or alcohol metabolism.

It is preferred in the hepatocytes that the expressions of alcohol or drug metabolism associated enzymes and antioxidant enzymes are increased. At this time, the alcohol metabolism associated enzyme is one or more enzymes selected from the group consisting of ADH1a, ADH1b, ADH1c, and CYP2E1, and the drug metabolism associated enzyme is one or more enzymes selected from the group consisting of CYP2C9, CYP2D6, and CYP3A4, and the antioxidant enzyme is either or both of catalase and Mn-SOD, but not always limited thereto.

The drug or alcohol above is preferably selected from the group consisting of ethanol, CCl₄, and acetaminophen, but not always limited thereto.

The system herein is preferably a kit or a chip, but not always limited thereto.

The human stem cell-derived hepatocytes of the present invention were confirmed to be similar in shape to human hepatocytes but displayed reduced expressions of drug or alcohol metabolism associated enzymes and antioxidant enzymes. Also from the result of the co-culture of human stem cell-derived hepatocytes with mouse hepatic stellate cells, it was confirmed that the alcohol dependent toxicity was reduced so that liver cell damage or apoptosis was reduced but the expressions of drug and alcohol metabolism associated enzymes and antioxidant enzymes were increased. Therefore, the human stem cell-derived hepatocytes co-cultured with hepatic stellate cells could be efficiently used for the evaluation system of drug or alcohol metabolism.

The present invention also provides a method for improving drug metabolism of human stem cell-derived hepatocytes comprising the step of treating retinoic acid to the hepatocytes differentiated from human stem cells *ex vivo.*

In the method above, the hepatocytes are preferably differentiated by the following steps, but not always limited thereto:
1) culturing human stem cells to induce the differentiation; and
2) culturing the differentiated cells above in a medium supplemented with hepatocyte growth factor.

In the method above, the human stem cells are preferably human embryonic stem cells or somatic cell derived induced pluripotent stem cells (iPS cells), but not always limited thereto.

In the method above, the retinoic acid is preferably secreted from hepatic stellate cells, and is preferably all trans retinoic acid or 9-cis retinoic acid, but not always limited thereto.

In the method above, it is preferred in the hepatocytes treated with retinoic acid that the expressions of alcohol or drug metabolism associated enzymes and antioxidant enzymes are increased. At this time, the alcohol metabolism associated enzyme is one or more enzymes selected from the group consisting of ADH1a, ADH1b, ADH1c, and CYP2E1, and the drug metabolism associated enzyme is one or more enzymes selected from the group consisting of CYP2C9, CYP2D6, and CYP3A4, and the antioxidant enzyme is either or both of catalase and Mn-SOD, but not always limited thereto.

In a preferred embodiment of the present invention, the present inventors induced the differentiation of human embryonic stem cells into hepatocytes. Then, the inventors compared the differentiated hepatocytes originated from human embryonic stem cells with human hepatocytes. As a result, it was confirmed that the hepatocytes differentiated from human embryonic stem cells were similar in cell shape (see Figure 1) but displayed different gene expression pattern. Precisely, the expression of drug or alcohol metabolism associated gene and anti-oxidation associated gene was significantly reduced (see Figure 2). The inventors also compared the gene expressions between adult hepatocytes (Adult-Hep) and fetal hepatocytes (Fetal-Hep) and between fetal hepatocytes and human embryonic stem cell-derived hepatocytes (hES-Hep). As a result, the expressions of those genes associated with alcohol metabolism were reduced in Fetal liver, compared with adult hepatocytes, and the expressions of those genes associated with alcohol metabolism were reduced more significantly in human embryonic stem cell-derived hepatocytes (hES-Hep) than in Fetal liver (see Figure 3).

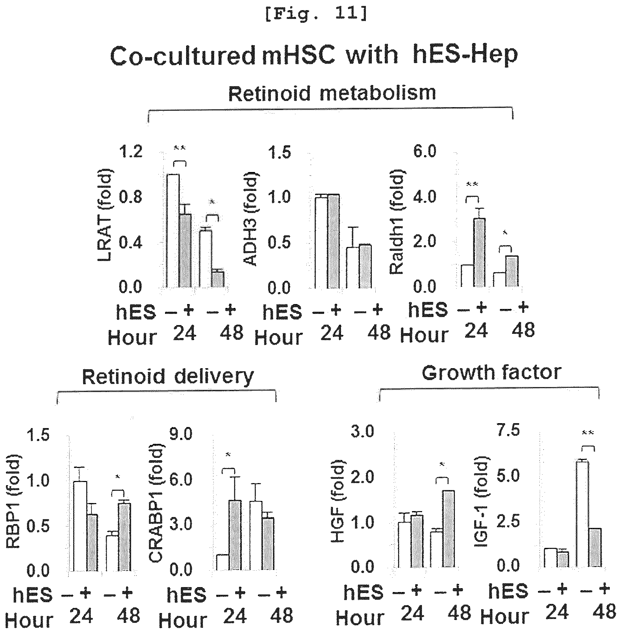

Mouse hepatic stellate cells were co-cultured with human embryonic stem cell-derived hepatocytes (see Figure 4). Then, gene expression pattern in the hepatic stellate cells was investigated. As a result, when the mouse hepatic stellate cells were co-cultured with human embryonic stem cell-derived hepatocytes, the expression of LRAT, the gene involved in retinol preservation, was reduced, but the expression of Raldh1 involved in retinoic acid synthesis was significantly increased. The expressions of RBP1 playing a role in carrying retinol to other cells and CRABP1 playing a role in carrying retinoic acid to other cells were significantly increased as well. However, the expressions of HGF and IGF-1 involved in the growth of hepatocytes were not changed much (see Figure 11).

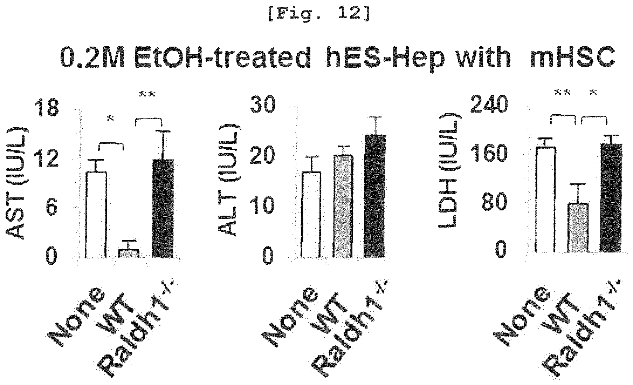

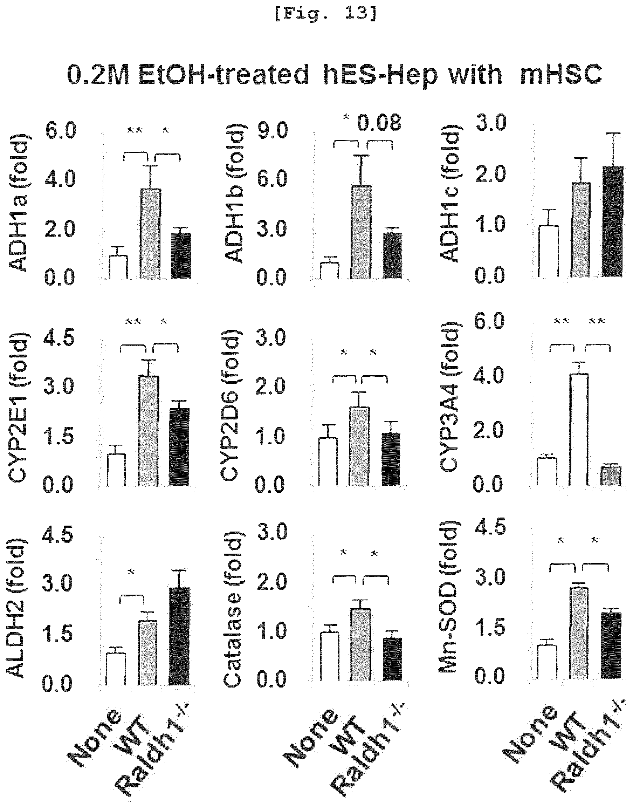

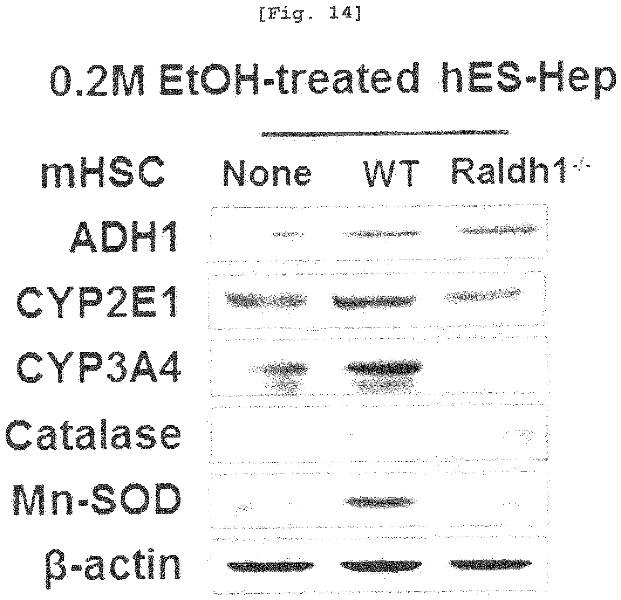

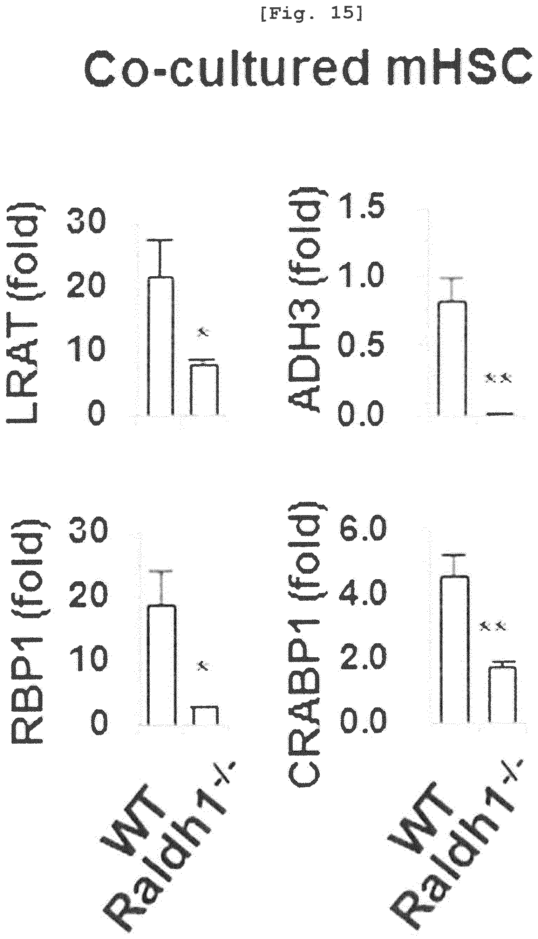

Human embryonic stem cell-derived hepatocytes (hES-Hep) were co-cultured with the mouse hepatic stellate cells (mHSCs) separated from the liver of a wild type (WT) mouse and a genetically engineered mouse with retinoic acid metabolism deficiency (Raldh1^{-/-}) so that it could not metabolize retinol into retinoic acid. Then, liver cell damage was observed. As a result, the levels of AST and LDH were significantly decreased in the medium where the mouse hepatic stellate cells separated from the wild type mouse were co-cultured. However, the levels of AST and LDH were not changed in the medium where the mouse hepatic stellate cells separated from the genetically engineered mouse (Raldh1^{-/-}) were co-cultured (see Figure 12). The expressions of alcohol metabolism associated enzymes, drug metabolism associated enzymes, and antioxidant enzymes were investigated. As a result, the expressions of those alcohol metabolism associated enzymes, drug metabolism associated enzymes, and antioxidant enzymes were increased when the mouse hepatic stellate cells separated from the wild type mouse were co-cultured. However, the expressions thereof were not changed when the mouse hepatic stellate cells separated from the genetically engineered mouse (Raldh1^{-/-}) were co-cultured (see Figures 13 and 14). The expressions of retinol associated genes were also investigated. As a result, when human embryonic stem cell-derived hepatocytes were co-cultured with the mouse hepatic stellate cells separated from the genetically engineered mouse (Raldh1^{-/-}), the expressions of LRAT and ADH3, the genes involved in retinol metabolism, RBP1 playing a role in retinol transportation, and CRABP1 playing a role in retinoic acid transportation were significantly reduced (see Figure 15).

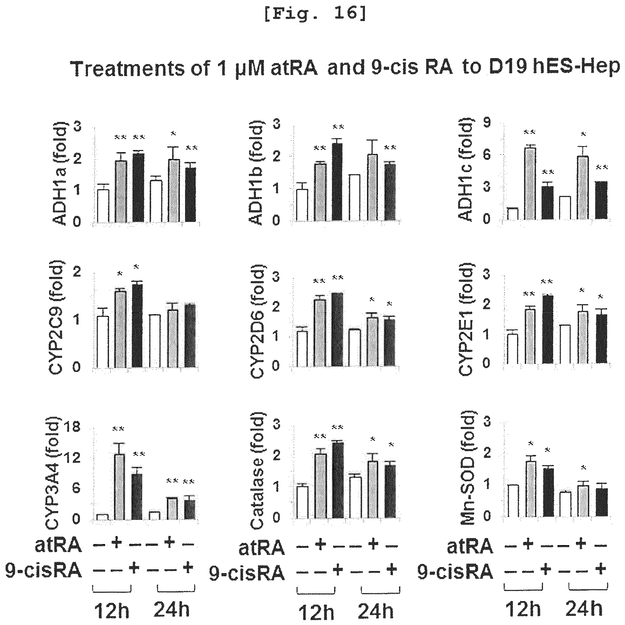

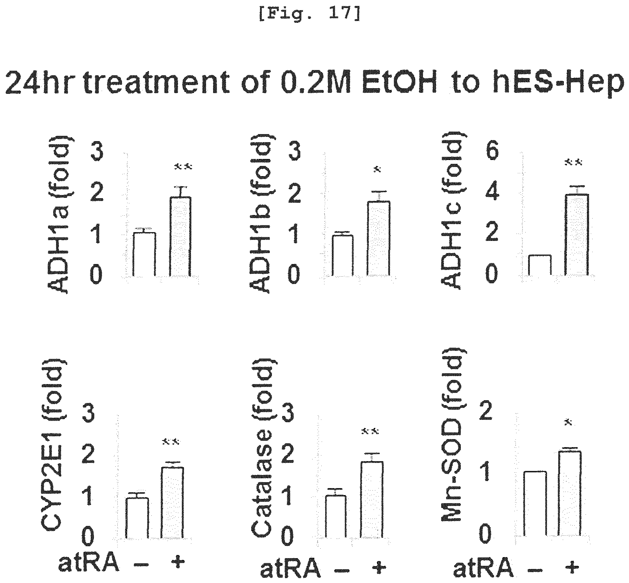

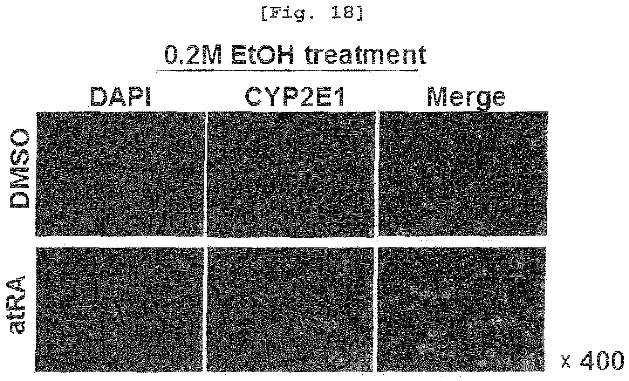

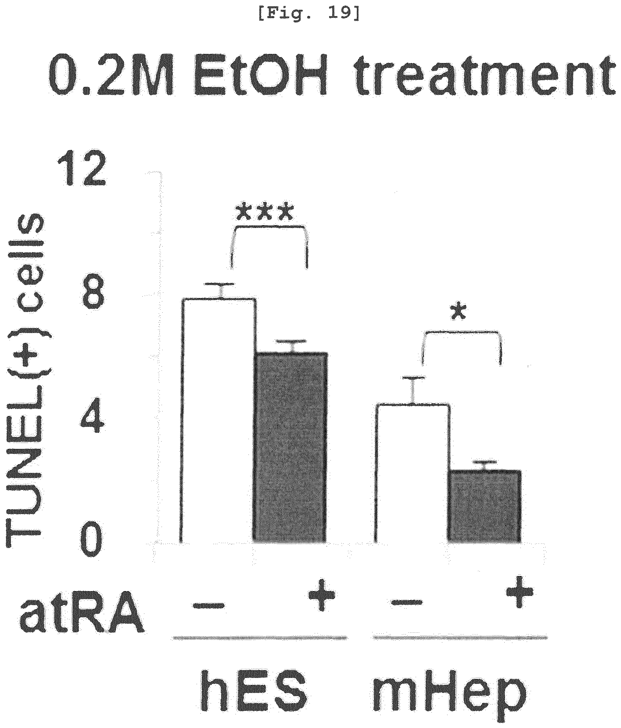

After treating human embryonic stem cell-derived hepatocytes with retinoic acid, the expressions of those genes involved in alcohol metabolism, drug metabolism, and anti-oxidation activity were investigated. As a result, when retinoic acid was treated to the cells, the expressions of those genes said above were all increased as shown when human embryonic stem cell-derived hepatocytes were co-cultured with the mouse hepatic stellate cells (see Figure 16). When ethanol was treated to the cells, the expressions of the enzymes associated with alcohol metabolism and anti-oxidation activity were increased (see Figure 17). In the meantime, the expression of CYP2E1, the alcohol metabolism associated enzyme, was investigated by immunofluorescence method. As a result, the expression of CYP2E1 was significantly increased in the human embryonic stem cell-derived hepatocytes treated with retinoic acid (see Figure 18). Apoptosis was also investigated. As a result, alcohol induced apoptosis was significantly reduced in the human embryonic stem cell-derived hepatocytes treated with retinoic acid (see Figure 19).

From the above results, it was confirmed that the expressions of the enzymes involved in drug and alcohol metabolism and anti-oxidation activity were increased in the human embryonic stem cell-derived hepatocytes (hES-Hep) treated with retinoic acid and accordingly toxicity was reduced owing to the increased alcohol metabolism. In conclusion, the inventors confirmed that the expressions of the enzymes associated with drug and alcohol metabolism and anti-oxidation activity were increased by retinoic acid.

Therefore, the human stem cell-derived hepatocytes of the present invention were confirmed to be similar in shape to human hepatocytes but displayed reduced expressions of drug or alcohol metabolism associated enzymes and antioxidant enzymes. From the result of the co-culture of human stem cell-derived hepatocytes with mouse hepatic stellate cells, it was confirmed that the expression pattern of retinol associated gene was changed. From the co-culture of human stem cell-derived hepatocytes with the mouse hepatic stellate cells isolated from the mouse with metabolic disorder so as not to metabolize retinol into retinoic acid, it was confirmed that liver cell damage was not reduced nor the expression patterns of drug and alcohol metabolism associated enzymes and anti-oxidant enzymes were not changed. In the meantime, when retinoic acid was treated to human stem cell-derived hepatocytes, the expressions of drug and alcohol metabolism associated enzymes and anti-oxidant enzymes were increased but apoptosis was decreased. Therefore, the method of co-culturing the hepatocytes differentiated from human stem cells above with hepatic stellate cells and the method for the treatment of retinoic acid to the hepatocytes differentiated from human stem cells were confirmed to be efficiently used as methods for improving drug metabolism function of the human stem cell-derived hepatocytes.

The present invention also provides the human stem cell-derived hepatocytes prepared by the method above.

The present invention further provides a method for evaluating drug or alcohol metabolism in hepatocytes comprising the following steps:
1) treating the said hepatocytes above with a test drug or alcohol *ex vivo;*
2) measuring the expression level of the enzyme related to the drug or alcohol metabolism in the hepatocytes of step 1); and
3) evaluating the drug or alcohol metabolism level by the expression level of the enzyme related to the drug or alcohol metabolism measured in step 2).

The human stem cells above are preferably human embryonic stem cells or somatic cell derived induced pluripotent stem cells, but not always limited thereto.

In the method above, it is preferred in the hepatocytes that the expressions of alcohol or drug metabolism associated enzymes and antioxidant enzymes are increased. At this time, the alcohol metabolism associated enzyme is one or more enzymes selected from the group consisting of ADH1a, ADH1b, ADH1c, and CYP2E1, and the drug metabolism associated enzyme is one or more enzymes selected from the group consisting of CYP2C9, CYP2D6, and CYP3A4, and the antioxidant enzyme is either or both of catalase and Mn-SOD, but not always limited thereto.

The test drug or alcohol above is preferably selected from the group consisting of ethanol, CCl₄, and acetaminophen, but not always limited thereto.

The human stem cell-derived hepatocytes of the present invention were confirmed to be similar in shape to human hepatocytes but displayed reduced expressions of drug or alcohol metabolism associated enzymes and antioxidant enzymes. From the result of the co-culture of human stem cell-derived hepatocytes with mouse hepatic stellate cells, it was confirmed that the expression pattern of retinol associated gene was changed. From the co-culture of human stem cell-derived hepatocytes with the mouse hepatic stellate cells isolated from the mouse with metabolic disorder so as not to metabolize retinol into retinoic acid, it was confirmed that liver cell damage was not reduced nor the expression patterns of drug and alcohol metabolism associated enzymes and anti-oxidant enzymes were not changed. In the meantime, when retinoic acid was treated to human stem cell-derived hepatocytes, the expressions of drug and alcohol metabolism associated enzymes and anti-oxidant enzymes were increased but apoptosis was decreased. Therefore, the method for the treatment of retinoic acid to the hepatocytes differentiated from human stem cells were confirmed to be efficiently used as a method for improving drug metabolism function of the human stem cell-derived hepatocytes.

The present invention also provides an evaluation system of drug or alcohol metabolism comprising the hepatocytes above.

The present invention also provides the hepatocytes above for the evaluation system of drug or alcohol metabolism.

It is preferred in the hepatocytes that the expressions of alcohol or drug metabolism associated enzymes and antioxidant enzymes are increased. At this time, the alcohol metabolism associated enzyme is one or more enzymes selected from the group consisting of ADH1a, ADH1b, ADH1c, and CYP2E1, and the drug metabolism associated enzyme is one or more enzymes selected from the group consisting of CYP2C9, CYP2D6, and CYP3A4, and the antioxidant enzyme is either or both of catalase and Mn-SOD, but not always limited thereto.

The drug or alcohol above is preferably selected from the group consisting of ethanol, CCl₄, and acetaminophen, but not always limited thereto.

The system herein is preferably a kit or a chip, but not always limited thereto.

The human stem cell-derived hepatocytes of the present invention were confirmed to be similar in shape to human hepatocytes but displayed reduced expressions of drug or alcohol metabolism associated enzymes and antioxidant enzymes. From the result of the co-culture of human stem cell-derived hepatocytes with mouse hepatic stellate cells, it was confirmed that the expression pattern of retinol associated gene was changed. From the co-culture of human stem cell-derived hepatocytes with the mouse hepatic stellate cells isolated from the mouse with metabolic disorder so as not to metabolize retinol into retinoic acid, it was confirmed that liver cell damage was not reduced nor the expression patterns of drug and alcohol metabolism associated enzymes and anti-oxidant enzymes were not changed. In the meantime, when retinoic acid was treated to human stem cell-derived hepatocytes, the expressions of drug and alcohol metabolism associated enzymes and anti-oxidant enzymes were increased but apoptosis was decreased. Therefore, the method for the treatment of retinoic acid to the hepatocytes differentiated from human stem cells were confirmed to be efficiently used as a method for improving drug metabolism function of the human stem cell-derived hepatocytes.

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Induction of the differentiation of human embryonic stem cells into hepatocytes

CHA15 (human embryonic stem cell line, CHA Research Institute) was maintained according to the standard protocol. The human embryonic stem cells were cultured with feeder cells on the plate coated with matrigel (BD biosciences) for 5 ∼ 6 days. The culture medium was RPMI 1640 (Welgene, Korea), which was replaced every 24 hours. The colony of the human embryonic stem cells was cut into 6 ∼ 9 pieces. The cell mass was placed on the plate coated with matrigel but not containing feeder cells, followed by culture in a 37°C incubator for 72 hours. It was the step of stabilization. After confirming the growth of the human embryonic stem cell colony, other cell fractions and culture fluid were eliminated therefrom by pipetting. The colony was differentiated into endoderm by using RPMI 1640 supplemented with 50X B27 (Gibco), and 50 ng/uL of activin A for 0 ∼ 5 days. The culture medium was replaced with fresh RPMI 1640 every 24 hours. The differentiated cells were further differentiated into hepatocytes in RPMI supplemented with 50X B27, 100 ng/uL of fibroblast growth factor-4, and 50 ng/uL of (bone morphogenetic protein-2 for 5 ∼ 10 days. Then, 50 ng/uL of hepatocyte growth factor (HGF; Peprotech) was added to high concentration culture fluid bulletkit, followed by culture for 10 ∼ 15 days. 50 ng/uL of oncostatin M and 1 mM dexametasone were added to the high concentration culture fluid, followed by culture for 15 ∼ 19 days to induce maturation of the hepatocytes. The culture medium in every differentiation stage was replaced every 24 hours. The primary human hepatocytes were maintained in DMEM containing a low concentration of glucose at 37°C with 5% CO₂ until use. To compare the function, the human hepatocytes (Lot no. Hu4246) obtained from a male at the age of 46 provided by Life Technologies Gibco (USA) as frozen were used.

### Example 2: Test animal care

The wild type (WT) male C57BL/6 mouse was purchased from Jackson Laboratory (USA). The mouse with metabolic disorder so as not to metabolize retinol into retinoic acid (Raldh1^{-/-}) was prepared by the conventional method reported earlier. The animal was taken care of in a germ free animal facility at KAIST (Korea Advanced Institute of Science and Technology). The animal was taken care of according to the guide for the care and use of laboratory animals provided by NIH (National Institute of Health). All the experimental stages with the animal were approved by animal care and use committee at KAIST.

### Example 3: Separation of hepatic stellate cells (mHSCs) from the test mouse

Hepatic stellate cells were separated from the wild type (WT) mouse and the Raldh1^{-/-} mouse of Example 2.

Particularly, a mouse at the body weight of 30 ∼ 34 g was anesthetized with 30 mg/kg of sodium pentobarbital via intraperitoneal injection. A tube was inserted in portal vein in a germ free condition. The liver was perfused with EGTA (ethylene glycol-bis(β-aminoethyl ester)*-N,N,N', N*'-tetraacetic acid) solution (5.4 mmol/L of KCl, 0.44 mmol/L of KH₂PO₄, 140 mmol/L of NaCl, 0.34 mmol/L of Na₂HPO₄, 0.5 mmol/L of EGTA, and 25 mmol/L of Tricine, pH 7.2) and HBSS (Hanks' Balanced Salt solution, Sigma-Aldrich, USA), followed by degradation with HBSS containing 0.075% type I collagenase (Warthington). For the separation, the liver cells were cultured on the plate coated with rat-tail collagen containing DMEM (Gibco-BRL) supplemented with 10% FBS until the confluence reached 60 ∼ 70%. To separate mouse hepatic stellate cells (mHSCs), EGTA solution was added to the mouse liver in situ, then the liver was perfused with perfusion buffer and lysis buffer (HBSS containing 0.02% DNase I and 0.009% collagenase type I) at 37°C for 20 ∼ 30 minutes. The obtained cell suspension was filtered by 70 um cell strainer, followed by centrifugation at room temperature for 5 minutes at 420 rpm to eliminate liver cells. The supernatant was washed and resuspended in HBSS, which was then loaded in 11.5% OptiPrep. Centrifugation was performed at 4°C for 15 minutes at 1400 g. Then, hepatic stellate cells were obtained from interface. The fat globule containing retinol was clearly observed in the isolated hepatic stellate cells.

### Experimental Example 1: Comparison between human embryonic stem cell-derived hepatocytes (hES-Hep) and human hepatocytes (Human-Hep)

### <1-1≥ Morphological comparison

The morphology was compared between the hepatocytes differentiated from human embryonic stem cells (hES-Hep) prepared by the same manner as described in Example 1 and human hepatocytes (human-Hep) by the observation under optical microscope.

As a result, as shown in Figure 1, the cell morphology of hES-Hep was very similar to that of Human-Hep (Figure 1).

### <1-2> Comparison of gene expression

The gene expression in the hepatocytes differentiated from human embryonic stem cells (hES-Hep) prepared by the same manner as described in Example 1 was compared with that of adult hepatocytes.

Particularly, RNA was extracted from the human embryonic stem cell-derived hepatocytes and the adult hepatocytes. Each chip was hybridized with each pool and illumina human HT-12(v3) beadchip was treated at KRIBB (Korea Research Institute of Bioscience & Biotechnology). Statistical analysis of the microarray was performed by using the free software R obtainable at http://www.r-project.org/. Heat map was made by standardizing the log gene expression level (0 indicates the standard error) by color-coding genewise. The diagram was made by using the matrix2png program (http://www.chibi.ubc.ca/matrix2png/) .

As a result, as shown in Figure 2, approximately 929 genes were comparatively down-regulated in the adult hepatocytes and the human embryonic stem cell-derived hepatocytes. In particular, those genes associated with drug or alcohol metabolism such as ADH1A, ADH1B, ADH1C, CYP1A2, CYP2C9, CYP2D6, CYP2E1, and CYP3A4, and the genes associated with anti-oxidation activity such as SOD2 and catalase were significantly down-regulated (Figure 2).

### <1-3> Comparison of drug or alcohol metabolism associated enzyme expression

Gene expression pattern was compared between adult-Hep and Fetal-Hep and between Fetal liver and hES-Hep, in order to compare the expression pattern of drug or alcohol metabolism associated enzymes.

Particularly, total RNA was extracted from the adult Hep, Fetal-Hep, and human embryonic stem cell-derived hepatocytes by using Trizol reagent (Invitrogen), followed by cDNA synthesis by using amfi-rivert II cDNA synthesis Master Mix (Gendepot) according to the manufacturer's protocol. Genes in interest were amplified from the transcript by using the primers listed in the Table 1 below by using SYBR green real-time PCR master mix (Toyobo, Japan). The real-time PCR was performed by using Bio-rad CFX96 real-time PCR detection system (Bio-rad). The gene expression level was calculated by Δ Δ Ct method and by using β-actin as a denominator of the gene expression. Compared with the control gene expression, the obtained value was presented as fold change.

**[Table 1]**

| Name | Size | Forward | Reverse |
|---|---|---|---|
| ADH1a | 102 | AGTCATCCCACTCGCTATTC C (SEQ. ID. NO: 1) | GTCCCCTGAGGATTGCTTAC A (SEQ. ID. NO: 2) |
| ADH1b | 224 | CCCGGAGAGCAACTACTGC (SEQ. ID. NO: 3) | AACCAGTCGAGAATCCACAG C (SEQ. ID. NO: 4) |
| ADH1c | 223 | CTCGCCCCTGGAGAAAGTC (SEQ. ID. NO: 5) | GGCCCCCAACTCTTTAGCC (SEQ. ID. NO: 6) |
| ALDH2 | 196 | TCGGCTACATCAACACGG (SEQ. ID. NO: 7) | TCTCCCAACAACCTCCTCT (SEQ. ID. NO: 8) |
| CYP2E1 | 67 | GCAAGAGATGCCCTACATGG A (SEQ. ID. NO: 9) | GGGCACGAGGGTGATGAA (SEQ. ID. NO: 10) |
| CYP2C9 | 137 | CCTCTGGGGCATTATCCATC (SEQ. ID. NO: 11) | ATATTTGCACAGTGAAACAT AGGA (SEQ. ID. NO: 12) |
| CYP2D6 | 289 | CTAAGGAACGACACTCATC AC (SEQ. ID. NO: 13) | CTCACCAGGAAAGCAAAGAC AC (SEQ. ID. NO: 14) |
| CYP3A4 | 78 | CAGGAGGAAATTGATGCAGT TTT (SEQ. ID. NO: 15) | GTCAAGATACTCCATCTGTA GCACAGT (SEQ. ID. NO: 16) |
| Catalase | 129 | GTTACTCAGGTGCGGGCATT CTAT (SEQ. ID. NO: 17) | GAAGTTCTTGACCGCTTTCT TCTG (SEQ. ID. NO: 18) |
| Mn-SOD | 369 | CTCCCCGACCTGCCCTACGA CTAC (SEQ. ID. NO: 19) | AAACCAAGCCAACCCCAACC TGAG (SEQ. ID. NO: 20) |
| b-actin | 285 | AGCGAGCATCCCCCAAAGTT (SEQ. ID. NO: 21) | GGGCACGAAGGCTCATCATT (SEQ. ID. NO: 22) |

As a result, as shown in Figure 3, it was confirmed from the comparison of gene expression between the adult hepatocytes and the fetal hepatocytes that the genes involved in alcohol metabolism were down-regulated in fetal hepatocytes and the down-regulation was more significant in human embryonic stem cell-derived hepatocytes than in fetal hepatocytes. Only if the human embryonic stem cell-derived hepatocytes were treated with alcohol, the alcohol metabolism associated enzymes were up-regulated (Figure 3).

### Experimental Example 2: Effect of the co-culture of mouse hepatic stellate cells with human embryonic stem cell-derived hepatocytes

### <2-1> Co-culture of mouse hepatic stellate cells with human embryonic stem cell-derived hepatocytes

Mouse hepatic stellate cells were co-cultured with human embryonic stem cell-derived hepatocytes.

Particularly, as shown in Figure 4, the human embryonic stem cell-derived hepatocytes obtained in Example 1 were co-cultured with the mouse hepatic stellate cells obtained in Example 3. 2 × 10⁵ mouse hepatic stellate cells were cultured on the plate (Corning, USA) with 8 µm holes coated with human embryonic stem cell-derived hepatocytes cultured in DMEM supplemented with 10% FBS. The cell ratio of mouse hepatic stellate cells to human embryonic stem cell-derived hepatocytes was maintained as 1:2. 24 hours later, the cells and the supernatant were obtained or the culture continued for another 24 hours after the treatment with 200 mM (0.2 M) ethanol (EtOH). 48 hours later, the cells and the medium were collected (Figure 4).

### <2-2> Investigation of liver cell damage after the co-culture of mouse hepatic stellate cells with human embryonic stem cell-derived hepatocytes

After the co-culture of mouse hepatic stellate cells with human embryonic stem cell-derived hepatocytes, the levels of AST (aspartate aminotransferase), ALT (alanine aminotransferase), and LDH (lactate dehydrogenase) were measured in order to investigate liver cell damage.

Particularly, mouse hepatic stellate cells were co-cultured with human embryonic stem cell-derived hepatocytes by the same manner as described in Experimental Example <2-1>. Then, the medium was collected and liver cell damage was investigated. The levels of AST, ALT, and LDH were analyzed by using the kit purchased from IDEX Laboratories (USA).

As a result, as shown in Figure 5, the levels of AST, ALT, and LDH in the human embryonic stem cell-derived hepatocytes were reduced simply by the co-culture with the mouse hepatic stellate cells for 24 hours. When the human embryonic stem cell-derived hepatocytes were treated with ethanol, the levels of AST, ALT, and LDH were all decreased (Figure 5). The results above seemed to be resulted from that alcohol was decomposed fast and accordingly alcohol induced toxicity was reduced and ROS (reactive oxygen species) was inhibited by the increase of anti-oxidation associated enzyme expression.

### <2-3> Investigation of apoptosis after the co-culture of human embryonic stem cell-derived hepatocytes with mouse hepatic stellate cells

Human embryonic stem cell-derived hepatocytes were co-cultured with mouse hepatic stellate cells. Then, TUNEL assay was performed to investigate apoptosis therein,.

Particularly, human embryonic stem cell-derived hepatocytes were co-cultured with mouse hepatic stellate cells by the same manner as described in Experimental Example <2-1>. Then, the cells were treated with ethanol, followed by fixation with 4% paraformaldehyde. The fixed cells were treated with 100% methanol (Merck, Millipore) for 15 minutes in order for them to be permeabilized. Apoptosis was investigated by using In Situ Cell Death Detection Kit (Fluorescence, Roche). Nucleus of the cell was stained with DAPI, followed by visualization using Leica DMI 4000B automatic vertical microscope system (Germany) equipped with Leica EL6000 and Leica CTR 4000 CCD camera. Computer assisted images were analyzed with Leica Application Suite Advanced Fluorescence (LAS AF, Leica).

As a result, as shown in Figure 6, it was confirmed that apoptosis was hardly observed in the normal mouse hepatocytes, while apoptosis was increased in the human embryonic stem cell-derived hepatocytes treated with alcohol. In the meantime, the embryonic stem cell-derived hepatocytes which had been co-cultured with mouse stellate cells displayed significantly reduced apoptosis (Figure 6). The results above indicate that the mouse stellate cells can increase alcohol metabolism of human embryonic stem cell-derived hepatocytes.

### <2-4> Investigation of gene expression pattern in human embryonic stem cell-derived hepatocytes co-cultured with mouse hepatic stellate cells

Human embryonic stem cell-derived hepatocytes were co-cultured with mouse hepatic stellate cells, followed by investigation of the expressions of alcohol metabolism associated enzymes, drug metabolism associated enzymes, and anti-oxidant enzymes.

Particularly, human embryonic stem cell-derived hepatocytes were co-cultured with mouse hepatic stellate cells by the same manner as described in Experimental Example <2-1>, and then the cells were treated with ethanol. Real-time PCR was performed by using the primers listed in Table 1 by the same manner as described in Experimental Example <1-3> in order to investigate the expression patterns of alcohol metabolism associated enzymes (ADH1b, ADH1c and CYP2E1), drug metabolism associated enzymes (CYP2C9, CYP2D6 and CYP3A4), and anti-oxidant enzymes **(catalase and Mn-SOD).**

As a result, as shown in Figure 7, it was confirmed that the expressions of the enzymes associated with alcohol metabolism (ADH1b, ADH1c and CYP2E1), the enzymes associated with drug metabolism (CYP2C9, CYP2D6 and CYP3A4), and the anti-oxidant enzymes (catalase and Mn-SOD) were all increased in the human embryonic stem cell-derived hepatocytes co-cultured with mouse hepatic stellate cells (Figure 7).

### <2-5> Investigation of protein expression pattern in human embryonic stem cell-derived hepatocytes co-cultured with mouse hepatic stellate cells

Human embryonic stem cell-derived hepatocytes were co-cultured with mouse hepatic stellate cells. Then, the expressions of alcohol metabolism associated enzymes, drug metabolism associated enzymes, and anti-oxidant enzymes were investigated.

Particularly, human embryonic stem cell-derived hepatocytes were co-cultured with mouse hepatic stellate cells by the same manner as described in Experimental Example <2-1>. Then, the cells were treated with ethanol. The cells were homogenized with RIPA buffer (30 mmol/L of Tris pH 7.5, 150 mmol/L of sodium chloride, 1 mmol/L of PMSF (phenylmethylsulfonyl fluoride), 1 mmol/L of sodium orthovanadate, 1% NP-40 (nonidet p-40), 10% glycerol, phosphatase inhibitor, and protease inhibitor) to extract protein. 50 ∼ 80 µg of the obtained protein was loaded on SDS-PAGE. The protein was then transferred onto nitrocellulose membrane. The protein expression was investigated by reacting the membrane with the primary antibodies such as anti-β-actin (Sigma-Aldrich), anti-α-CYP2E1 (Millipore), anti-CYP3A4, anti-catalase (Cell signaling, USA), anti-ADH1, and anti-Mn-SOD (Santa-Cruz) antibodies. Alkaline phosphate conjugated anti-rabbit antibody was used as the secondary antibody. The protein expression was examined by using PhosphoImager (GE Healthcare, USA) and ECL detection system.

As a result, as shown in Figure 8, it was confirmed that the expressions of ADH1, CYP2E1, CYP3A4, catalase, and Mn-SOD were all increased in the human embryonic stem cell-derived hepatocytes which had been co-cultured with mouse hepatic stellate cells (Figure 8).

### <2-6> Immunocytochemistry with human embryonic stem cell-derived hepatocytes co-cultured with mouse hepatic stellate cells

Human embryonic stem cell-derived hepatocytes were co-cultured with mouse hepatic stellate cells, followed by immunofluorescence to investigate the expression of CYP2E1, the alcohol metabolism associated enzyme.

Particularly, human embryonic stem cell-derived hepatocytes were co-cultured with mouse hepatic stellate cells by the same manner as described in Experimental Example <2-1>. The cells were treated with ethanol and then fixed with 4% paraformaldehyde. The fixed cells were treated with 100% methanol (Merck, Millipore) for 15 minutes in order for them to be permeabilized. Blocking was performed by treating the cells with 5% goat serum at 37°C for 1 hour. The cells were stained with anti-CYP2E1 antibody (Millipore, Germany). Nucleus of the cell was stained with DAPI, followed by visualization using Leica DMI 4000B automatic vertical microscope system (Germany) equipped with Leica EL6000 and Leica CTR 4000 CCD camera. Computer assisted images were analyzed with Leica Application Suite Advanced Fluorescence (LAS AF, Leica).

As a result, as shown in Figure 9, it was confirmed that the expression of CYP2E1 was significantly increased in the human embryonic stem cell-derived hepatocytes co-cultured with mouse hepatic stellate cells (Figure 9).

### <2-7> Investigation of the activity of anti-oxidant enzyme in human embryonic stem cell-derived hepatocytes co-cultured with mouse hepatic stellate cells

Human embryonic stem cell-derived hepatocytes were co-cultured with mouse hepatic stellate cells. Then, the activities of catalase and Mn-SOD, the anti-oxidant enzymes, were investigated.

Particularly, human embryonic stem cell-derived hepatocytes were co-cultured with mouse hepatic stellate cells by the same manner as described in Experimental Example <2-1>. Then, the enzyme activities of catalase and Mn-SOD were examined. The enzyme activities were measured by using a specific enzyme activity analyzing kit. The catalase analysis kit and the SOD activity analysis kit (Colorimetric, Abcam) were used according to the manufacturer's protocol.

As a result, as show in Figure 10, it was confirmed that the activities of catalase and Mn-SOD were increased in the human embryonic stem cell-derived hepatocytes co-cultured with mouse hepatic stellate cells (Figure 10).

Based on the above results, it was confirmed that the method of co-culturing the hepatocytes with mouse hepatic stellate cells was effective in increasing the expressions of drug metabolism and alcohol metabolism associated enzymes in human embryonic stem cell-derived hepatocytes.

### <2-8> Investigation of gene expression pattern in human embryonic stem cell-derived hepatocytes co-cultured with mouse hepatic stellate cells

Human embryonic stem cell-derived hepatocytes were co-cultured with mouse hepatic stellate cells. Then, the gene expression pattern was investigated.

Particularly, human embryonic stem cell-derived hepatocytes were co-culture with mouse hepatic stellate cells by the same manner as described in Experimental Example <2-1>. And real-time PCR was performed by using the primers listed in Table 2 by the same manner as described in Experimental Example <3-1>. Then, the expressions of retinoid metabolism associated genes (LRAT, ADH3, and Raldh1), retinoid migration associated genes (RBP1 and CRABP1), and growth factor genes (HGF and IGF-1) were investigated.

**[Table 2]**

| Name | Size | Forward | Reverse |
|---|---|---|---|
| B-actin | 148 | AGAGGGAAATCGTGCGTGA C (SEQ. ID. NO: 21) | CAATAGTGATGACCTGGCC GT (SEQ. ID. NO: 22) |
| LRAT | 370 | CTGACCAATGACAAGGAAC GCACTC (SEQ. ID. NO: 23) | CTAATCCCAAGACAGCCGA AGCAAGA (SEQ. ID. NO: 24) |
| ADH3 | 215 | GTGGGAGTAGCTGCTTCAG G (SEQ. ID. NO: 25) | GTGCATCAGATCAAAGGCT TG (SEQ. ID. NO: 26) |
| ALDH1A1 | 248 | GCTCAAGACAGTCGCAATG A (SEQ. ID. NO: 27) | TGGATAAGAACTGGGTCA CA (SEQ. ID. NO: 28) |
| Rbp1 (cellular) | 155 | CAGTGTGTGCAGAAGGAG A (SEQ. ID. NO: 29) | AGGTTATCTCCTCGGCTG T (SEQ. ID. NO: 30) |
| Crabp1 | 223 | CTTACTGGACCCGAGAGCT G (SEQ. ID. NO: 31) | GGAAAATGGGGTTGCCTAA T (SEQ. ID. NO: 32) |
| HGF (Hepatocyte growth factor) | 236 | TTCCCAGCTGGTCTATGGT C (SEQ. ID. NO: 33) | TGGTGCTGACTGCATTTCT C (SEQ. ID. NO: 34) |
| IGF-1 | 220 | CACATCATGTCGTCTTCAC ACC (SEQ. ID. NO: 35) | GGAAGCAACACTCATCCAC AATG (SEQ. ID. NO: 36) |

As a result, as shown in Figure 11, it was confirmed that the expression of LRAT, the gene involved in retinol preservation, was reduced but the expression of Raldh1, the gene involved in retinoic acid synthesis, was significantly increased in the human embryonic stem cell-derived hepatocytes co-cultured with mouse hepatic stellate cells. It was also confirmed that the expression of RBP1 carrying retinol to other cells and the expression of CRABP1 carrying retinoic acid to other cells were significantly increased but the expressions of HGF and IGF-1 involved in liver cell growth were not changed in the human embryonic stem cell-derived hepatocytes co-cultured with mouse hepatic stellate cells (Figure 11).

From the above results, it was confirmed that retinol preservation in hepatic stellate cells was decreased but instead retinol was metabolized into retinoic acid by Raldh1, which was conjugated with CRABP1 and then migrated into human embryonic stem cell-derived hepatocytes so that it reacted with retinoic acid nucleus receptor to affect the expressions of metabolism associated enzymes in the hepatocytes.

### Experimental Example 3: Confirmation of the importance of retinoic acid metabolism in human embryonic stem cell-derived hepatocytes co-cultured with mouse hepatic stellate cells

### <3-1> Investigation of liver cell damage in human embryonic stem cell-derived hepatocytes co-cultured with mouse hepatic stellate cells

Human embryonic stem cell-derived hepatocytes were co-cultured with the mouse hepatic stellate cells separated from the liver of a wild type (WT) mouse and a genetically engineered mouse with retinoic acid metabolism deficiency (Raldh1^{-/-}) so that it could not metabolize retinol into retinoic acid. Then, the levels of AST (aspartate aminotransferase), ALT (alanine aminotransferase), and LDH (lactate dehydrogenase) were measured to confirm liver damage.

Particularly, mouse hepatic stellate cells were isolated from a mouse by the same manner as described in Example 3. Human embryonic stem cell-derived hepatocytes were co-cultured with the prepared mouse hepatic stellate cells by the same manner as described in Experimental Example <2-1> for 24 hours. The cells were treated with 0.2 M EtOH, followed by further culture for 24 hours. The medium was collected and liver cell damage was measured. The levels of AST, ALT, and LDH were analyzed by using a kit purchased from IDEXX Laboratories (USA).

As a result, as shown in Figure 12, it was confirmed that the levels of AST and LDH were significantly reduced in the medium where the hepatic stellate cells isolated from a wild type mouse were co-cultured. However, the levels of AST and LDH were not decreased in the medium where the hepatic stellate cells isolated from a Raldh1 knocked-out mouse were co-cultured (Figure 12).

### <3-2> Investigation of gene expression pattern in human embryonic stem cell-derived hepatocytes co-cultured with mouse hepatic stellate cells

Human embryonic stem cell-derived hepatocytes were co-cultured with the mouse hepatic stellate cells separated from the liver of a wild type (WT) mouse and a genetically engineered mouse with retinoic acid metabolism deficiency (Raldh1^{-/-}) so that it could not metabolize retinol into retinoic acid. Then, the gene expressions of alcohol metabolism associated enzymes, drug metabolism associated enzymes, and anti-oxidant were investigated.

Particularly, mouse hepatic stellate cells were isolated from a mouse by the same manner as described in Example 3. Human embryonic stem cell-derived hepatocytes were co-cultured with the prepared mouse hepatic stellate cells by the same manner as described in Experimental Example <2-1> for 24 hours. The cells were treated with ethanol. Then, real-time PCR was performed with the ethanol treated cells by using the primers listed in Table 1 by the same manner as described in Experimental Example <1-3> to investigate the expressions of alcohol metabolism associated enzymes (ADH1a, ADH1b, ADH1c, ALDH2, and CYP2E1), drug metabolism associated enzymes (CYP2D6 and CYP3A4), and anti-oxidant enzymes (catalase and Mn-SOD).

As a result, as shown in Figure 13, it was confirmed that the expressions of alcohol and drug metabolism associated enzymes and anti-oxidant enzymes were increased in the human embryonic stem cell-derived hepatocytes co-cultured with the mouse hepatic stellate cells separated from the wild type mouse but the expressions of alcohol and drug metabolism associated enzymes and anti-oxidant enzymes were hardly observed in the human embryonic stem cell-derived hepatocytes co-cultured with the mouse hepatic stellate cells separated from the Raldh1 knocked-out mouse (Figure 13).

### <3-3> Investigation of protein expression in human embryonic stem cell-derived hepatocytes co-cultured with mouse hepatic stellate cells

Human embryonic stem cell-derived hepatocytes were co-cultured with the mouse hepatic stellate cells separated from the liver of a wild type (WT) mouse and a genetically engineered mouse with retinoic acid metabolism deficiency (Raldh1^{-/-}) so that it could not metabolize retinol into retinoic acid. Then, the protein expressions of alcohol metabolism associated enzymes, drug metabolism associated enzymes, and anti-oxidant were investigated.

Particularly, mouse hepatic stellate cells were isolated from a mouse by the same manner as described in Example 3. Human embryonic stem cell-derived hepatocytes were co-cultured with the prepared mouse hepatic stellate cells by the same manner as described in Experimental Example <2-1>. Then, the cells were treated with ethanol. The cells were homogenized with RIPA buffer (30 mmol/L of Tris pH 7.5, 150 mmol/L of sodium chloride, 1 mmol/L of PMSF (phenylmethylsulfonyl fluoride), 1 mmol/L of sodium orthovanadate, 1% NP-40 (nonidet p-40), 10% glycerol, phosphatase inhibitor, and protease inhibitor) to extract protein. 50 ∼ 80 µg of the obtained protein was loaded on SDS-PAGE. The protein was then transferred onto a nitrocellulose membrane. The protein expression was investigated by reacting the membrane with the primary antibodies such as anti-β-actin (Sigma-Aldrich), anti-α-CYP2E1 (Millipore), anti-CYP3A4, anti-catalase (Cell signaling, USA), anti-ADH1, and anti-Mn-SOD (Santa-Cruz) antibodies. Alkaline phosphate conjugated anti-rabbit antibody was used as the secondary antibody. The protein expression was examined by using PhosphoImager (GE Healthcare, USA) and ECL detection system.

As a result, as shown in Figure 14, it was confirmed that the protein expressions of CYP2E1, CYP3A4, and Mn-SOD were significantly increased in the human embryonic stem cell-derived hepatocytes co-cultured with the mouse hepatic stellate cells separated from the wild type mouse, while those protein expressions were not increased in the human embryonic stem cell-derived hepatocytes co-cultured with the mouse hepatic stellate cells separated from the Raldh1 knocked-out mouse (Figure 14).

### <3-4> Investigation of the retinol associated gene expression in human embryonic stem cell-derived hepatocytes co-cultured with mouse hepatic stellate cells

Human embryonic stem cell-derived hepatocytes were co-cultured with the mouse hepatic stellate cells separated from the liver of a wild type (WT) mouse and a genetically engineered mouse with retinoic acid metabolism deficiency (Raldh1^{-/-}) so that it could not metabolize retinol into retinoic acid. Then, the retinol associated enzyme gene expression was investigated.

Particularly, mouse hepatic stellate cells were isolated from a mouse by the same manner as described in Example 3. Human embryonic stem cell-derived hepatocytes were co-cultured with the prepared mouse hepatic stellate cells by the same manner as described in Experimental Example <2-1>. Then, the cells were treated with ethanol. Real time PCR was performed with the cells by the same manner as described in Experimental Example <1-3> in order to investigate the gene expressions of the enzymes associated with retinol metabolism (LRAT and ADH3), retinol movement (RBP1), and retinoic acid movement (CRABP1) .

As a result, as shown in Figure 15, it was confirmed that the gene expressions of the enzymes associated with retinol metabolism (LRAT and ADH3), retinol movement (RBP1), and retinoic acid movement (CRABP1) were significantly reduced in the human embryonic stem cell-derived hepatocytes co-cultured with the mouse hepatic stellate cells separated from the Raldh1 knocked-out mouse (Figure 15).

### Experimental Example 4: Effect by the treatment of retinoic acid

### <4-1> Gene expression after the treatment of retinoic acid

Human embryonic stem cell-derived hepatocytes were treated with retinoic acid, followed by investigation of the gene expressions of alcohol metabolism associated enzymes, drug metabolism associated enzymes, and anti-oxidant enzymes.

Particularly, the human embryonic stem cell-derived hepatocytes differentiated by the same manner as described in Example 1 were treated with 1 ump all-trans retinoic acid or 9-cis-retinoic acid, followed by culture for 12 ∼ 24 hours. Then, real time PCR was performed by the same manner as described in Experimental Example <1-3> in order to examine the expressions of alcohol metabolism associated enzymes (AHD1a, ADH1b, ADH1c, and CYP2E1), drug metabolism associated enzymes (CYP2C9, CYP2D6, and CYP3A4), and anti-oxidant enzymes (catalase and Mn-SOD).

As a result, as shown in Figure 16, it was confirmed that the expressions of various enzymes said above were all increased simply by the treatment of retinoic acid just as shown when the hepatocytes were co-cultured with mouse hepatic stellate cells (Figure 16) .

### <4-2> Gene expression after the treatment of retinoic acid and ethanol

Human embryonic stem cell-derived hepatocytes were treated with retinoic acid and ethanol, followed by investigation of the gene expressions of alcohol metabolism associated enzymes, drug metabolism associated enzymes, and anti-oxidant enzymes.

Particularly, the human embryonic stem cell-derived hepatocytes differentiated by the same manner as described in Example 1 were treated with 1 ump all-trans retinoic acid and 0.2 M EtOH, followed by culture for 24 hours. Then, real time PCR was performed by the same manner as described in Experimental Example <1-3> in order to examine the expressions of alcohol metabolism associated enzymes (AHD1a, ADH1b, ADH1c, and CYP2E1) and anti-oxidant enzymes (catalase and Mn-SOD).

As a result, as shown in Figure 17, it was confirmed that the expressions of alcohol metabolism associated enzymes and anti-oxidant enzymes were all increased when retinoic acid and ethanol were treated (Figure 17).

### <4-3> Immunocytochemistry after the treatment of retinoic acid

Human embryonic stem cell-derived hepatocytes were treated with retinoic acid, followed by immunofluorescence in order to examine the expression of CYP2E1, the alcohol metabolism associated enzyme.

Particularly, the human embryonic stem cell-derived hepatocytes differentiated by the same manner as described in Example 1 were treated with 1 ump all-trans retinoic acid and 0.2 M EtOH, followed by culture for 24 hours. The cells were fixed with 4% paraformaldehyde. The fixed cells were treated with 100% methanol (Merck, Millipore) for 15 minutes in order for them to be permeabilized. Blocking was performed by treating the cells with 5% goat serum at 37°C for 1 hour. The cells were stained with anti-CYP2E1 antibody (Millipore, Germany). Nucleus of the cell was stained with DAPI, followed by visualization using Leica DMI 4000B automatic vertical microscope system (Germany) equipped with Leica EL6000 and Leica CTR 4000 CCD camera. Computer assisted images were analyzed with Leica Application Suite Advanced Fluorescence (LAS AF, Leica).

As a result, as shown in Figure 18, it was confirmed that the expression of CYP2E1 was significantly increased in the human embryonic stem cell-derived hepatocytes treated with retinoic acid (Figure 18).

### <4-4> Investigation of apoptosis after the treatment of retinoic acid

Human embryonic stem cell-derived hepatocytes were treated with retinoic acid. Then, TUNEL assay was performed to investigate apoptosis therein.

Particularly, the human embryonic stem cell-derived hepatocytes differentiated by the same manner as described in Example 1 were treated with 1 uM all-trans retinoic acid and 0.2 M EtOH, followed by culture for 24 hours. The cells were fixed with 4% paraformaldehyde. The fixed cells were treated with 100% methanol (Merck, Millipore) for 15 minutes in order for them to be permeabilized. Apoptosis was investigated by using In Situ Cell Death Detection Kit (Fluorescence, Roche). Nucleus of the cell was stained with DAPI, followed by visualization using Leica DMI 4000B automatic vertical microscope system (Germany) equipped with Leica EL6000 and Leica CTR 4000 CCD camera. Computer assisted images were analyzed with Leica Application Suite Advanced Fluorescence (LAS AF, Leica).

As a result, as shown in Figure 19, it was confirmed that the apoptosis induced by alcohol was significantly reduced in the human embryonic stem cell-derived hepatocytes treated with retinoic acid (Figure 19).

From the above results, it was confirmed that the expressions of drug and alcohol metabolism associated enzymes and anti-oxidant enzymes were increased in the human embryonic stem cell-derived hepatocytes treated with retinoic acid, indicating that alcohol metabolism was accordingly accelerated to inhibit the toxic effect. Therefore, the increase of the expressions of those liver enzymes associated with drug and alcohol metabolism and anti-oxidation was confirmed.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended Claims.

## Claims

1. A method for improving drug metabolism of human stem cell-derived hepatocytes comprising the step of co-culturing the hepatocytes differentiated from human stem cells with hepatic stellate cells (HSCs) *ex vivo.*

2. The method for improving drug metabolism of human stem cell-derived hepatocytes according to claim 1, wherein the hepatocytes are characteristically differentiated by the following steps:
1) culturing human stem cells to induce the differentiation; and
2) culturing the differentiated cells above in a medium supplemented with hepatocyte growth factor.

3. The method for improving drug metabolism of human stem cell-derived hepatocytes according to claim 1, wherein the human stem cells are characteristically human embryonic stem cells or somatic cell derived induced pluripotent stem cells (iPS cells).

4. The method for improving drug metabolism of human stem cell-derived hepatocytes according to claim 1, wherein the hepatic stellate cells are characteristically mouse originated hepatic stellate cells.

5. The method for improving drug metabolism of human stem cell-derived hepatocytes according to claim 1, wherein the ratio of hepatic stellate cells to human stem cell-derived hepatocytes for the co-culture is characteristically 1:2.

6. The method for improving drug metabolism of human stem cell-derived hepatocytes according to claim 1, wherein the cultured hepatocytes characteristically display the increased expressions of alcohol or drug metabolism associated enzymes and anti-oxidant enzymes.

7. The method for improving drug metabolism of human stem cell-derived hepatocytes according to claim 6, wherein the alcohol metabolism associated enzyme is one or more enzymes selected from the group consisting of ADH1a, ADH1b, ADH1c, and CYP2E1, and the drug metabolism associated enzyme is one or more enzymes selected from the group consisting of CYP2C9, CYP2D6, and CYP3A4, and the antioxidant enzyme is either or both of catalase and Mn-SOD.

8. A human stem cell-derived hepatocyte prepared by the method of claim 1.

9. A method for evaluating drug or alcohol metabolism in hepatocytes comprising the following steps:
1) treating the hepatocytes of claim 8 with a test drug or alcohol ex *vivo;*
2) measuring the expression level of the enzyme related to the drug or alcohol metabolism in the hepatocytes of step 1); and
3) evaluating the drug or alcohol metabolism level by the expression level of the enzyme related to the drug or alcohol metabolism measured in step 2).

10. An evaluation system of drug or alcohol metabolism comprising the hepatocytes of claim 8.

11. A method for improving drug metabolism of human stem cell-derived hepatocytes comprising the step of treating the hepatocytes differentiated from human stem cells with retinoic acid *ex vivo.*

12. The method for improving drug metabolism of human stem cell-derived hepatocytes according to claim 11, wherein the hepatocytes are characteristically differentiated by the following steps:
1) culturing human stem cells to induce the differentiation; and
2) culturing the differentiated cells above in a medium supplemented with hepatocyte growth factor.

13. The method for improving drug metabolism of human stem cell-derived hepatocytes according to claim 11, wherein the human stem cells are characteristically human embryonic stem cells or somatic cell derived induced pluripotent stem cells (iPS cells).

14. The method for improving drug metabolism of human stem cell-derived hepatocytes according to claim 11, wherein the retinoic acid is characteristically secreted in hepatic stellate cells.

15. The method for improving drug metabolism of human stem cell-derived hepatocytes according to claim 11, wherein the retinoic acid is characteristically all trans retinoic acid or 9-cis retinoic acid.

16. The method for improving drug metabolism of human stem cell-derived hepatocytes according to claim 11, wherein the hepatocytes treated with retinoic acid characteristically display the increased expressions of alcohol or drug metabolism associated enzymes and anti-oxidant enzymes.

17. The method for improving drug metabolism of human stem cell-derived hepatocytes according to claim 16, wherein the alcohol metabolism associated enzyme is one or more enzymes selected from the group consisting of ADH1a, ADH1b, ADH1c, and CYP2E1, and the drug metabolism associated enzyme is one or more enzymes selected from the group consisting of CYP2C9, CYP2D6, and CYP3A4, and the antioxidant enzyme is either or both of catalase and Mn-SOD.

18. A human stem cell-derived hepatocyte prepared by the method of claim 11.

19. A method for evaluating drug or alcohol metabolism in hepatocytes comprising the following steps:
1) treating the hepatocytes of claim 18 with a test drug or alcohol *ex vivo;*
2) measuring the expression level of the enzyme related to the drug or alcohol metabolism in the hepatocytes of step 1); and
3) evaluating the drug or alcohol metabolism level by the expression level of the enzyme related to the drug or alcohol metabolism measured in step 2).

20. An evaluation system of drug or alcohol metabolism comprising the hepatocytes of claim 18.

21. A use of the hepatocytes of claim 8 for the evaluation system of drug or alcohol metabolism.

22. A use of the hepatocytes of claim 18 for the evaluation system of drug or alcohol metabolism.
